# EUROPEAN PATENT SPECIFICATION

(11) **EP 1 469 941 B1**
(45) Date of publication and mention of the grant of the patent: **31.10.2007**
(21) Application number: 03700841.4
(22) Date of filing: 07.01.2003
(51) Int. Cl.: B01J 13/02, A61K 9/50, C01B 31/02, C09C 3/06

(54) **MICROPARTICLES AND METHODS FOR THEIR PRODUCTION**
MIKROPARTIKEL UND VERFAHREN ZU IHRER HERSTELLUNG
MICROPARTICULES ET LEURS PROCEDES DE PRODUCTION

(30) Priority: 07.01.2002 GB 0200259
(43) Date of publication of application: 27.10.2004
(73) Proprietor: THE UNIVERSITY OF READING, Reading RG6 2AH (GB)
(72) Inventor: TSANG, S. C., Dep. of Chemistry, Univ. of Reading, Berkshire RG6 6AD (GB); CHAN, Henry, Bo, So, Manchester, Greater Manchester M34 5SD (GB)
(74) Representative: Paget, Hugh Charles Edward
(86) International application number: PCT/GB2003/000029
(87) International publication number: WO 2003/057359

(56) References cited:
- WO-A-02/00192
- US-A- 5 472 749
- US-A- 6 009 457
- US-A1- 2001 051 766
- GADD G E ET AL: "The encapsulation of Ni in graphitic layers using C60 as a precursor" CARBON, XX, XX, vol. 39, no. 12, October 2001 (2001-10), pages 1769-1787, XP004299747 ISSN: 0008-6223

## Description

### FIELD OF THE INVENTION

This invention relates to microparticles having metal-containing cores encapsulated in coatings in the form of shells of graphite, and to methods of making such microparticles.

### BACKGROUND TO THE INVENTION

There has been great interest in the incorporation of foreign materials into enclosed nano-carbon cages. This interest has been driven by the potential applications of these filled carbon capsules, which lie in areas as diverse as optical, electronic, storage, magnetic recording materials, and nuclear medicine. In particular, carbon (onion-shell) structures of extreme strength may offer excellent protection to their encapsulated nanomaterials for applications. In addition, the close packing structures of the carbon shells do not allow exchange of molecules/materials from inner cores to the external environment. We have now found that materials of extremely reactive (air sensitive) or hazardous (radioactive) nature can be safely caged in the carbon-enclosed structures.

The idea of using carbon caged structures as molecular containers dates back many years [1,2] before the actual discovery of fullerene and related structures. Research in this area is chiefly driven by the potential applications of filled nanocapsules in areas such as electronics (quantum dots and wires), magnetic data storage, xerographics, lubrication, sensors and medicinal materials [3-5]. So far, several groups have succeeded in encapsulating various nanosized materials into fullerene structures by either using Krätschmer-Huffman arc [6,7], laser [8] or electron irradiation methods [9] during the fullerene synthesis or by creating an 'opening' in the carbon caged structures using chemical methods [10-12] prior to filling. Yields of the endohedral metallofullerenes synthesis [8] tend to be low and there are doubts about stability. Filled polygonal shaped carbon nanoparticles and nanotubes appear to be more promising from the point of view of applications. The techniques currently used to synthesise these structures include modifications of Krätschmer-Huffman arc method and methods involving heat treatment of impregnated microporous carbon (13,14). It is noted that there is no macroscopic synthetic method yet developed for the preparation of filled carbon onions (quasi-spherical graphitic shells) although minuscule amounts were produced through irradiation of amorphous carbon, nanotubes or nanoparticles with foreign atoms with an intense electron beam [9]. No reported work has been found on the preparation of total carbon encapsulation of radioactive nanoparticles.

US-A-6,099,457 discloses radioactive implants having a radioactive material enclosed in an inorganic shell.

US-A-2001 051766 is a document relating to medical instrumentation. This document discloses that radioisotopes can be held within carbon structures.

### SUMMARY OF THE INVENTION

By this invention, a novel methodology for the encapsulation of radioactive elements within the carbon cage is provided. This simple technique allows production of macroscopic quantities of quasi-spherical graphitic/fullerenic shells (carbon onions) that can encapsulate nanoparticles containing radioactive element(s) of a very narrow range of particle diameters. We believe the method may offer new routes for safe handling/disposal of radioactive substances and the carbon coated nano-radioactive products may find applications in medical imaging or radiotherapy.

According to the invention in a first aspect, there is provided a method of making microparticles as set out in claim 1.

The basis of the invention in this aspect are the findings that stable colloidal particles can be produced containing metal-oxo species of Fe, Co, Ni or Pd, and that these particles can be pyrolyzed to provide a metal-containing core, typically of metal, alloy, carbide or oxide or mixtures of these, encapsulated in a good quality graphitic shell which contains hetero atoms which are believed to cause curvature to the carbon-atom layers of the graphite. Further it has been found that a second metal, such as a radionuclide, can be incorporated in the colloidal particles. The first metal, which is at least one of Fe, Co, Ni and Pd, is believed to play a role in catalyzing the formation of the graphite during pyrolysis. The hetero atom, which should be chosen to become chemically bound in the graphite molecular structure, may be selected from N, B, P, S and O, and is most preferably N. The hetero atom is believed to cause curvature of the carbon layers of the graphitic shell, resulting in an encapsulating coating having few or no defects or fractures.

The metal-oxo species in the colloidal particle is typically insoluble in the medium which contains it. It may be produced by oxidation of a soluble compound of the first metal. Two methods of carrying this out are proposed.

In one particular method, step (i) is performed by forming a solution of a compound of the first metal in the liquid medium which is a polar solvent and heating the solution in the presence of the surfactant and an oxidizing agent to convert the compound to the metal-oxo species and form the colloidal particles. In this method, a high boiling point polar solvent is preferably employed, e.g. one having a boiling point above 100°C, or even above 200°C. The oxidizing agent may be for example oxygen or a compound of a second metal which becomes incorporated in the colloidal particles. Such a compound of a second metal is for example an oxo-anion, such as ReO₄⁻ or TcO₄⁻.

In a second method, step (i) is performed by forming an emulsion having dispersed phase droplets stabilized by the surfactant and containing a dissolved compound of the first metal and oxidizing the compound of the first metal to produce the metal-oxo species and form the colloidal particles. The dispersed phase droplets may include a dissolved oxidizing agent in the form of a compound of a second metal, as described above.

The colloidal particle includes source material of carbon and the hetero atom, for the formation of the graphitic shell. Thus it is not required that another carbon source, e.g. a gas, is provided during the pyrolysis. The carbon may effect reduction of the metal species, to provide metal or alloy as the core. The carbon and hetero atom may be present in a complex of the first metal. Preferably this metal complex includes ligands selected from cyanide, isocyanide, cyanate and isocyanate.

In a second aspect, the invention provides microparticles as set out in claim 11. Graphitic shell contains chemically bound hetero atoms which as mentioned above are believed to cause the graphitic carbon layers of the shell to be curved.

Preferably, the metal-containing core contains, in addition to at least one of Fe, Co, Ni and Pd, a radionuclide.

In nuclear medicine, the choice and form of radionuclides should be carefully chosen. The choice of a radionuclide for imaging purposes is chiefly dictated by the necessity of minimizing the radiation dose to the patient and the detection characteristics of present-day nuclear medicine instrumentation. The forms should preferably be non-toxic in the desired amounts, and would not directly involve in the patient's physiological body mechanisms. The form, structure and morphology and concentration of the radioactive material will also affect the imaging quality (small particle size will give better imaging quality i.e. smaller pixels). This invention describes a novel, easy, convenient method for the synthesis of complete carbon encapsulated radionuclides with a narrow particle size distribution suitable for diagnostic imaging and therapeutic applications. The impermeable carbon coatings offer total isolation of the enclosed nuclides from the environment.

The novel method described herein is concerned with a methodology for the production of a complete carbon encapsulated radioactive material with a small range of particle size. We believe these materials produced from the method will find applications in lung ventilation and lung perfusion imaging and other diagnostic and radiotherapy areas.

This method may be suitable to encapsulate a wide range of radionuclides (parental or daughter nuclides) with different emitted radiation and decay times.

| | | | | | |
|---|---|---|---|---|---|
| Typical example, | (i) | ⁹⁹Mo → | ^{99m}Tc → | ⁹⁹Tc → | ⁹⁹Ru |
| Half-life: | | 67 hours | 6 hours | long | stable |
| | (ii) | ¹¹³Sn → | ^{113m}Sn → | ¹¹³In | |
| Half-life | | 115 days | 1.67 hours | stable | |
| | (iii) | ⁶⁸Ge → | ⁶⁸Ga → | ⁶⁸Zn | |
| Half-life | | 275 days | 1.1 hours | stable | |
| | (iv) | ⁸¹Rb → | ^{81m}Kr → | ⁸¹Kr | |
| Half-life | | 4.7 hours | 13 seconds | stable | |

Technetium-99m (^{99m}Tc) has excellent physical characteristics for diagnostic imaging having a half-life of 6 h and emitting gamma-ray photons at an energy of 140 keV which are suitable for detection with a gamma camera. ^{99m}Tc is easily obtained from a commercially available ⁹⁹Mo/^{99m}Tc generator. ⁹⁹Mo is a fission product which is obtainable in a carrier-free form with a high specific activity. It has a half-life of 66.02 h and decays to ^{99m}TC by β- emission, which decays by isomeric transition to ⁹⁹Tc. In commercially available generators ⁹⁹Mo is absorbed onto an alumina column. Elution of the generator with 0.9% sodium chloride solution elutes the ^{99m}Tc in the form of sodium pertechnetate (Na^{99m}TcO₄) leaving the ⁹⁹Mo bound to the columnl¹⁵. ⁹⁹Mo/^{99m}Tc generators are generally purchased by hospitals on a weekly basis and have a working life of one week. ^{99m}Tc is incorporated in a variety of chemical forms for oral intake, injection or for lung ventilation/perfusion.

For lung imaging "Technegas" has been developed. It is an ultrafine ^{99m}TC-labelled aerosol introduced by W. Burch [22] in 1986. The small radioactive particles (nanometric in size) in a Technegas aerosol result in better clinical images than those obtained from other radioactive aerosols. It is known that the size and the content of the Tc particle in the aerosol can critically affect the quality of the images. The size of the aerosol particles also determines the site of lung deposition (bronchial and alveolar regions), subsequent modes and rates of clearance and hence will affect the diagnostic information obtained. More recent work, using electron microscopy, has shown that ^{99m}TC particles in Technegas are in fact relatively large crystals of over 100 nm in diameter. Another problem that arises with the production of Technegas is that significant quantities of ^{99m}Tc are incompletely protected in the carbon matrix. This leads to leaching of radioactive ^{99m}Tc to body fluids hence resulting in the undesirable appearance of ^{99m}Tc in the saliva, oesophagus, stomach and thyroid gland and thus degrading the quality of the images. In addition the high cost of purchasing the commercial Technegas generator has limited its use in many centres.

Our method in one embodiment involves the preparation of graphitic-encapsulated microparticles, more particularly nanoparticles, by the dissolution of a source or sources of iron, carbon and a hetero atom (usually N, but B, P, S or O for example may be used) in a polar solvent at elevated temperature; partially decomposing the iron compound to an insoluble iron oxygen species; size controlling and stabilizing the partially oxidised iron species by use of a surfactant; and pyrolyzing the stabilized species to yield a metallic core encapsulated by a graphitic/hetero shell. To form a radionuclide-containing particle, a salt or complex of a radionuclide is also present. It is found that during the partial decomposition of the iron compound, especially in the absence of oxygen, the radionuclide is incorporated into the core.

Since the graphitic encapsulation derives primarily from the carbon source, regulation of the carbon:iron ratio can help determine the numbers of encapsulating layers around a core; this in turn may act as a modulator or regulator of the radiation emitted from a radionuclide in the core.

In more detail some of the most important synthesis steps in obtaining the desired products have been elucidated from study of an iron cyanide complex as a starting material. These include the fact that iron species (catalyst) catalyse formation of enclosed graphitic structure from its attached cyanide ligand (or carbon and nitrogen containing ligand, stabiliser) upon heating, which will subsequently enclose the iron particle forming completely encapsulated particles. Prior to heat treatment, control in particle size is important in order to make encapsulated nanosized particles. We therefore show that adding surfactants or polymers controls the particle sizes and stabilisation of insoluble iron and cyanide-containing nanoparticles when soluble iron cyanide species is partially decomposed in a high-boiling polar solvent. This is based on the fact that the polymer/surfactant adsorbs at the surface of the newly formed nano-particles forming micellar protection so preventing aggregation. In other contexts, stabilisation of colloid in a polymer solution is a well-established technique typically in paint and inkjet technologies. Thus, we show that controlled size of Fe cyanide containing nanoparticles stabilised with adsorbed polymers/surfactants is a most important step before the radioactive element incorporation. The incorporation of foreign element(s) to the stabilised iron containing particles can be subsequently performed by developing specific interactions (chemical linkage, ion-exchange, redox attachment, etc) with the Fe(II) containing particles. Finally, after the removal of solvents followed by heat treatment the stabilised nanoparticles containing iron and foreign atoms with a narrow size distribution will give rise to the desired products. It is noted that the residue cyanide species in the particles will provide the carbon source for the formation of enclosed graphitic structure. We show that Re and Tc species can be successfully incorporated into the carbon-encapsulated iron containing nanoparticles via using ReO₄⁻ or TcO₄⁻. Incorporation of ^{99m}Tc is thus possible. Re and Tc have almost identical chemical properties (due to the lanthanide contraction). Other radioactive elements such as ^{99m}Mo, ¹¹³Sn can similarly be incorporated into this novel carrier (surfactant stabilised iron(oxo) cyanide nanoparticles) by employing similar chemistry.

The invention thus provides a low temperature solution method for encapsulation of radioactive nano-particles/clusters with a fine control in particle size. There are many advantages in the safe handling/disposal of radioactive materials if the materials are stored in an impervious carbon coating. Early work suggested it was difficult to carry out complete encapsulation of material with graphitic carbon unless the temperature rose above the vaporisation temperatures of graphite (>2500°C) during electric arc excitation [22]. However, there are many disadvantages associated with the extremely high temperature synthetic method especially regarding to the difficulty in controlling the particle size. Complete carbon encapsulation over radioactive nanoparticles thus may require a high temperature since amorphous carbon atoms do not offer total protection to these particle against leaching. The crystallisation of amorphous carbon atoms into complete graphitic protective capsule structures (graphitic carbon shells) is known to take place at minimum temperature of 2500°C [25]. However, the alternative would be to induce graphitisation at low temperatures by the use of catalyst. The phenomenon of low temperature (<700°C) "catalytic graphitisation" from a different variety of carbon sources over Fe, Co, Ni, Cr, Pd, has been known for many years. Here we demonstrate clearly that the formation of iron or iron carbide encapsulated in carbon shells from heat treatments of stabilised iron (oxo) cyanide species can be achieved. The cyanide species provide carbon source for the formation of enclosed graphitic shells. This indicates an effective catalytic carbonisation over the iron materials.

In summary, we report the (a) effectiveness of using catalytic component (Fe or alternatively Ni, Co or Pd, e.g. as cyanides) to form well-defined sized nanoparticles in a high boiling polar solvent (with stabiliser) during their cyanide decomposition; (b) incorporation of foreign atoms (radioactive atom, such as ^{99m}Tc) into the iron oxo-cyanide nanoparticle aggregates can be achieved using the oxidative ^{99m}TcO₄ ions through a fast redox trapping mechanism; (c) thermal decomposition of these colloidal particles (with cyanide) result in the enclosed graphitic structure embracing radioactive element(s).

### BRIEF INTRODUCTION OF THE DRAWINGS

In the drawings:
Figure 1 is an electron micrograph showing a large discrete iron-containing particle encapsulated within multi-layer graphitic carbon shell prepared from decomposition of iron cyanide particle in nitrogen (scale bar = 50 nm);
Figure 2 shows the powder X-ray diffraction (XRD) spectrum of pure Fe^{III}₄[Fe^{II}(CN)₆]₃ salt acting as a precursor;
Figures 3 and 4 show the powder X-ray diffraction (XRD) spectra of partial air decomposed Fe^{III}₄[Fe^{II}(CN)₆]₃ in refluxing dioctylether (Figure 3: small amount of dissolved oxygen in dioctylether; Figure 4: oxygen continuously purging through the system);
Figure 5 (a TEM micrograph) shows the colloidal stable iron-oxo-cyanide containing nanoparticles obtained via refluxing 0.86 g Fe^{III}₄[Fe^{II}(CN)₆]₃ salt in 40 ml dioctylether solvent with addition of oleic acid (20 ml). Small but homogeneous sized organic stabilised nanoparticles (- 10 nm) are clearly visible (nanosize particles giving a super-lattice packing);
Figure 6a is a typical high resolution transmission electron micrograph (lattice image) showing iron nanoparticles with the interplanar spacing of 0.21 ± 0.05 nm corresponding to (111) of fcc Fe (cementite) encapsulated in concentric quasi-spherical graphitic shells of 3.4 x 10⁻¹⁰ m spacing (scale bar = 5 nm);
Figure 6b is a TEM showing the same product as Figure 6a on a larger scale (scale bar = 1 nm);
Figure 7 is the EDX spectrum of Re-incorporated stabilised Fe nanoparticles which show the selected area rich in iron and rhenium (Cu peaks arise from the copper grids used for support);
Figure 8 shows the corresponding high-resolution TEM image of Re-incorporated stabilised Fe nanoparticles with very well defined size and shape (as Figure 7) (scale bar = 20 nm);
Figure 9a is a high resolution TEM image of a spherical graphitic shell structure (2-5 layers) filled with Fe and Re containing nanoparticles after applying heat treatments to the particles of Figure 8 (scale bar = 20 nm);
Figure 9b is a high resolution TEM showing the same product as Figure 9a on a larger scale (scale bar = 5 nm), with inset EDX spectrum of the selected area showing Re and Fe in the particle core (the Cu peaks arise from copper support grids);
Figure 10 shows the result of irradiation of amorphous carbon with intense electron beam in TEM showing it to have spherical graphitic concentric shells (onions); and a diagrammatic model attached (scale bar 100 x 10⁻¹⁰ m);
Figure 11 is a high resolution TEM showing a spherical graphitic shell structure filled with a nanoparticle containing Fe and ^{99m}Tc produced in Example 4; and
Figure 12 is a bar chart of activity counts obtained in the procedure described in Example 4.

### Experimental Details and Results

### Example 1: Synthesis of Carbon Encapsulated Iron Particles (comparative)

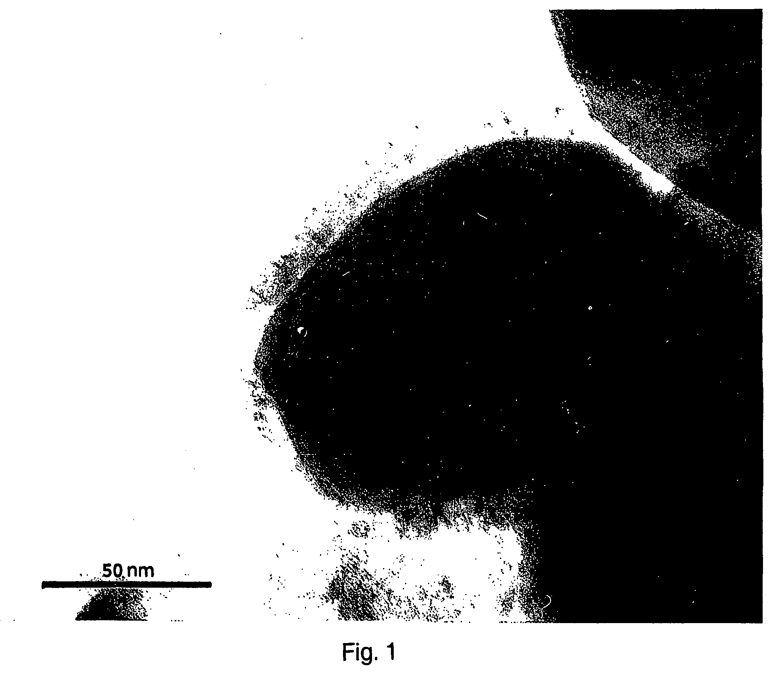

A sample of iron (III) ferrocyanide (purity >99.9%) obtained from Sigma plc, was transferred into a quartz tube with one end plugged with quartz wool. The filled tube, which was placed in the tube furnace, was first heated to 200°C (heating rate: 5°C/min, duration: 2h), and then finally to 900°C (heating rate: 10°C/min; duration: 3h) in N₂ atmosphere (approx. flow rate: 0.9 1/min). The black materials formed were stored in a sample vial. Elemental analyses from the EDX analysis and microanalysis showed that the sample after the heat treatment contained mainly C, Fe and a small amount of nitrogen (2-4%), which was scratched off the tube and examined by TEM. The TEM micrograph in Figure 1 shows that an iron-containing particle (iron and iron carbides) is encapsulated by graphitic carbon shells (onion structure) though the sizes of most of these encapsulated particles are very large (>0.1µm). Thus, it is clear that iron catalyses formation of enclosed graphitic structure from cyanide- or carbon-nitrogen-containing ligand. Other metals such as alkali or alkaline earths are unable to catalyse these enclosed graphitic structures from their cyanide salts, nor are iron species without using nitrogen containing salt/ligand/stabiliser.

### Example 2: Synthesis of Carbon Encapsulated Iron Nanoparticles:

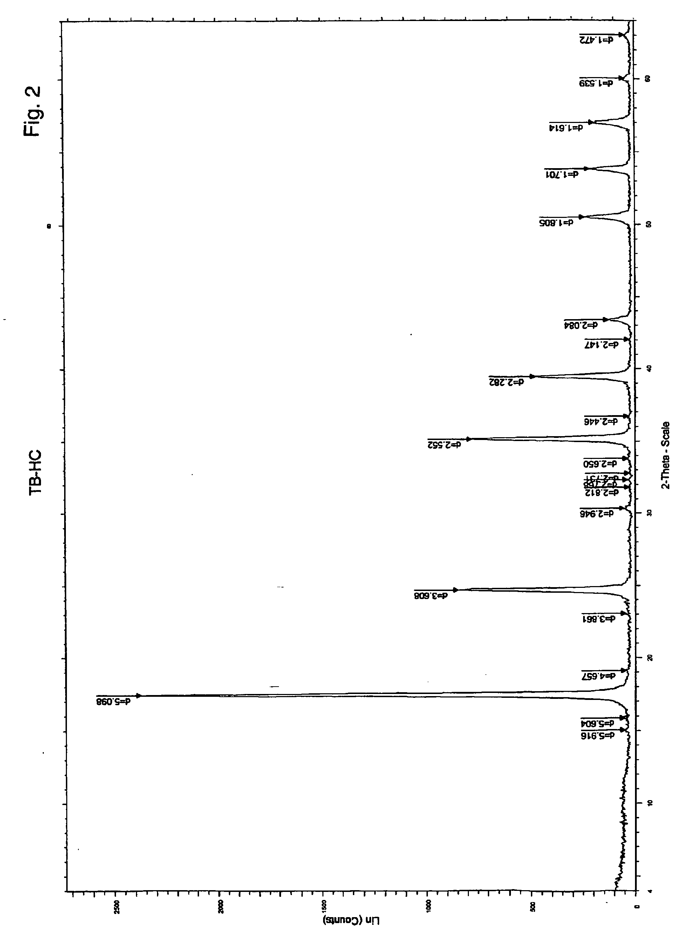

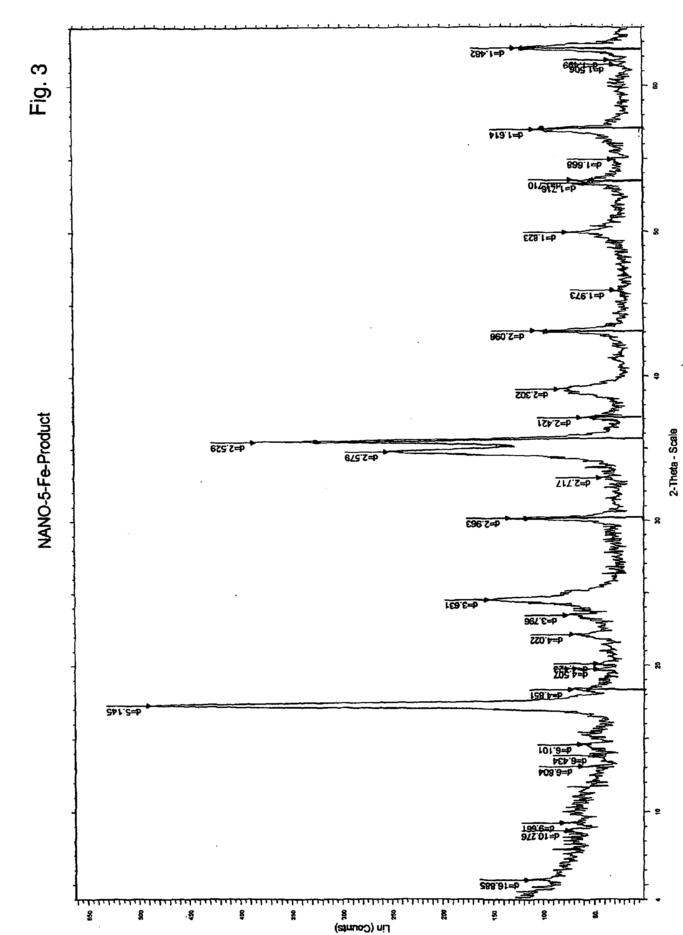

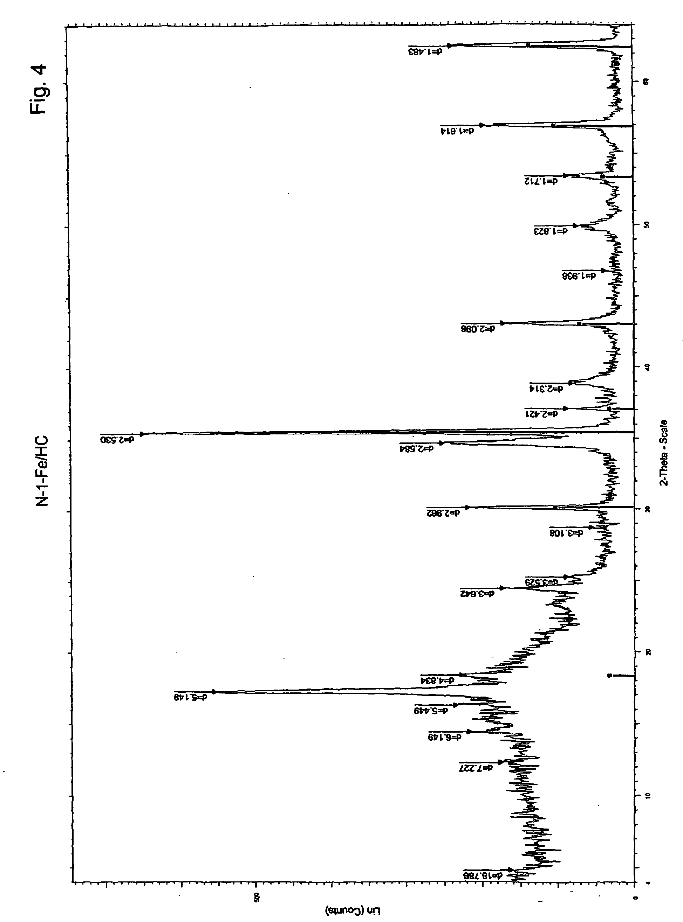

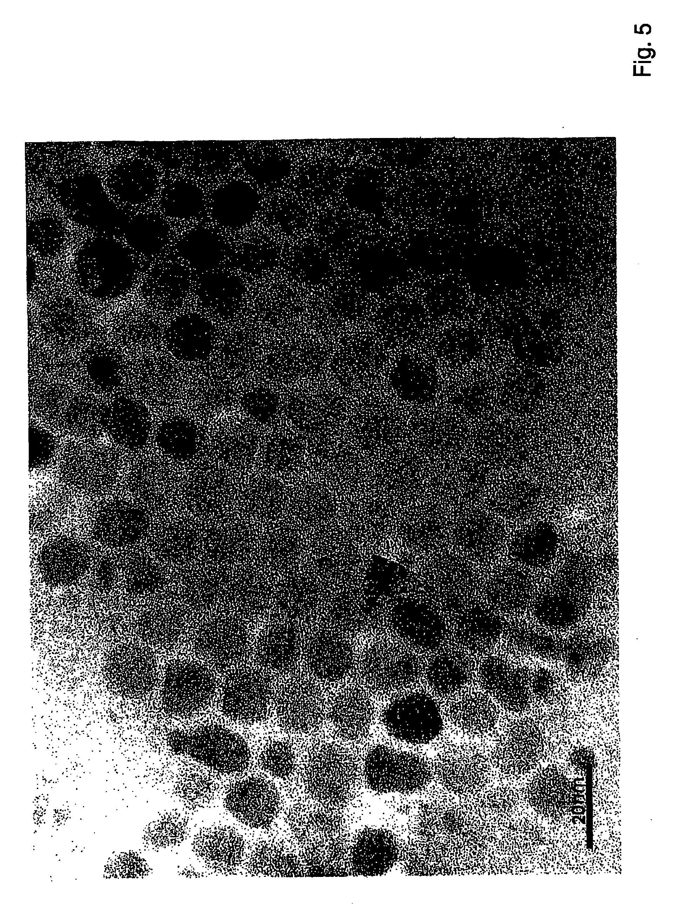

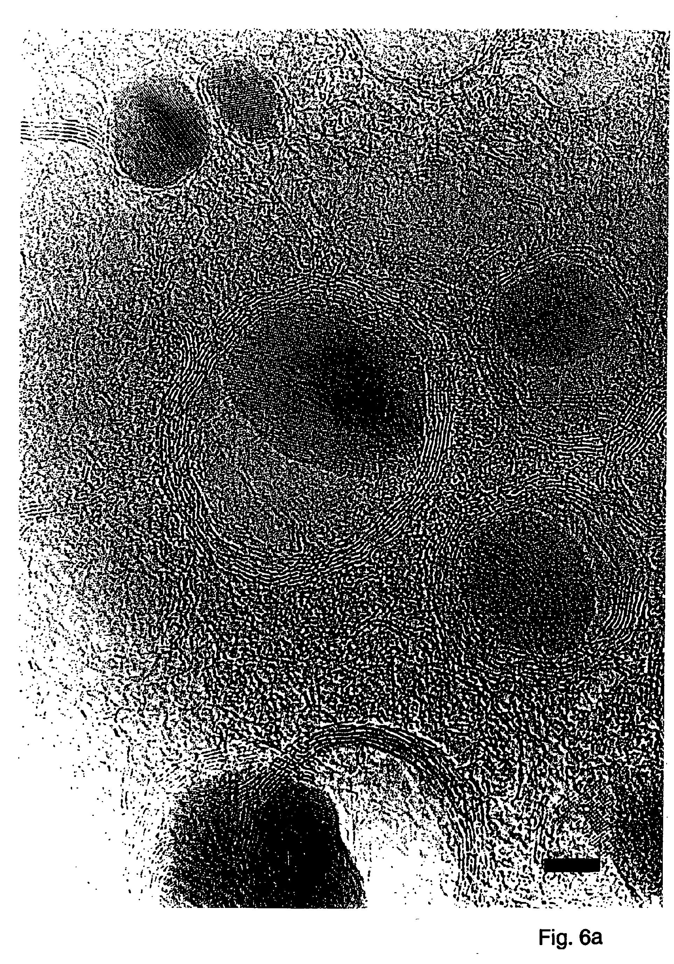

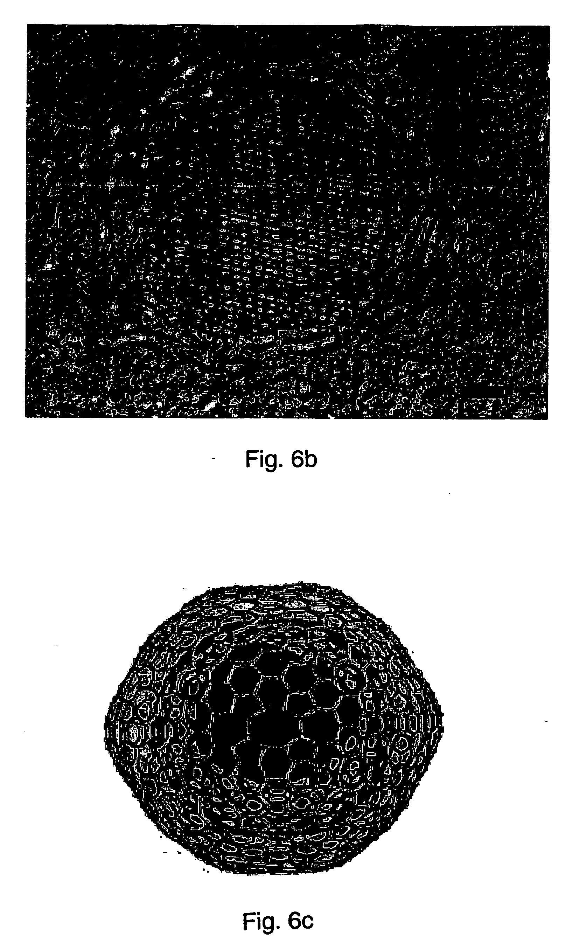

Example 1 discloses the use of iron cyanide salt for the formation of an enclosed graphitic structure embracing an iron particle, but no control in particle size was achieved. Here we describe the use of a polar high boiling solvent (such as dioctyl ether of boiling point 287°C) to dissolve the iron ferrocyanide compound at refluxing temperature (the compound is fairly soluble in the solvent at elevated temperature) giving intense blue colour solution. Iron-containing cyanide compounds are known to decompose at about 200-250°C [23,24]. In the presence of dissolved oxygen (air) achieved by purging the system with an air stream, the Fe^{II}(CN)₆⁴⁻ of the Fe^{III}₄[Fe^{II}(CN)₆]₃ salt decomposes to Fe(CN)₂₋₃O₁₋₂ⁿ⁻ losing the cyanide species but simultaneously replacing them with oxygen species [23]. This will ultimately lead to precipitating of iron oxide that is insoluble in the solvent. Figure 2 shows the powder X-ray diffraction (XRD) of the pure Fe^{III}4 [Fe^{II} (CN)₆]₃ salt. Iron oxide structure (magnetite, Fe₃O₄) is formed from the partial iron cyanide decomposition by air, giving mixed phases in the XRD patterns. The extent of the decomposition depends on the oxygen availability and duration of the treatment (Figures 3 and 4). An organic surfactant/stabiliser, such as oleic acid or polyvinylpyrrolidone (PVP), stabilises the partially oxidised iron cyanide species colloidally against precipitation from the solution. Figure 5 (TEM micrograph) shows the colloidal stable iron-oxo-cyanide containing nanoparticles obtained via refluxing 0.86g Fe^{III}₄[Fe^{II}(CN)₆]₃ salt in 40 ml dioctylether solvent with adding oleic acid (20 ml). Small but homogeneous particle size (nanosize particles giving a super-lattice packing) is obtained using this preparative mixture. There is a clear indication that the size of the particle can also be finely controlled by tailoring the ratio of oleic acid to the iron compound. Oleic acid, having a polar head group (acid group) and a double bond in the middle of the molecule, acts as surfactant. It is envisaged that the double bond will interact strongly with the iron species in the inner core of a micelle while its polar head will face outwards forming a normal phase micelle in this polar solvent. Hence the micellar structure provides a fine control in particle size. Application of the same heat treatment as mentioned in Example 1 to the colloidal iron containing particles of this example produces iron/iron carbide particle encapsulated in the enclosed graphitic carbon shelled structure (see Figures 6a and 6b and compare the model view in Figure 6c).

### Example 3: Synthesis of Carbon Encapsulated Nanoparticles containing Iron and Rhenium:

Example 2 shows that molecular oxygen from air can partially oxidise the iron cyanide compound in dioctylether at elevated temperatures. Adding a small quantity of sodium perrhenate (inorganic oxidant) to the mixture, as described in Table 1, can also oxidise the iron (II) cyanide species. Experimental details for typical synthesis are described below:

**Table 1. The starting materials. All the materials were used as supplied by Aldrich.**

| **Chemical Name** | **Chemical Formula** | **Mol.Wt.** | **Act. Wt/g** | **Features** |
|---|---|---|---|---|
| Sodium Perrhenate | NaReO₄ | 273 | **0.28** | White Crystals |
| Iron (III) Ferrocyanide | Fe^{III}₄ [Fe^{II} (CN)₆]₃ | 859.25 | **0.86** | Blue powder |
| Dioctylether | [CH₃(CH₂)₇]₂O | 242.45 | **40 ml** | Clear Liquid |
| Oleic acid | CH₃(CH₂)₇CH=CH(CH₂)₇COOH | 282 | **20 ml** | - |

The chemicals shown above, see Table 1, were mixed in a 3-necked round bottom flask using a magnetic stirrer bar. Before the mixture was heated up to 290°C for 18h, a gentle stream of nitrogen gas was bubbled directly into the mixture for 30 min. Nitrogen gas was continuously bubbled into mixture during the reaction. The black mixture formed was allowed to cool to room temperature. To separate the particles, 20 ml of ethanol was added to the product before the resultant mixture was centrifuged at 4000 rpm for 20 min. This procedure was repeated 3 times. The blue resultant solids were allowed to air dry in the fume cupboard.

Carbon encapsulation is achieved by heating the particles in nitrogen as follows. The as-synthesised sample was first transferred into a quartz tube with one end plugged with quartz wool. The filled tube, which was placed in the tube furnace, was first heated to 200°C (heating rate: 5°C/min, duration: 2h), and then finally to 900°C (heating rate: 10°C/min; duration: 3h) in N₂ atmosphere (approx. flow rate: 0.9 1/min). The black materials were stored in a sample vial.

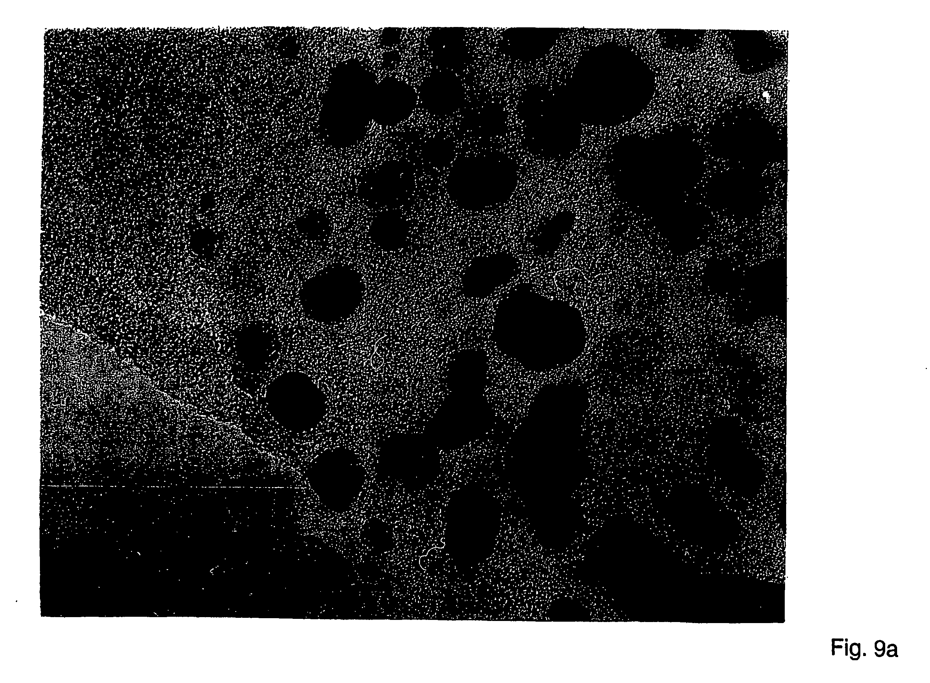

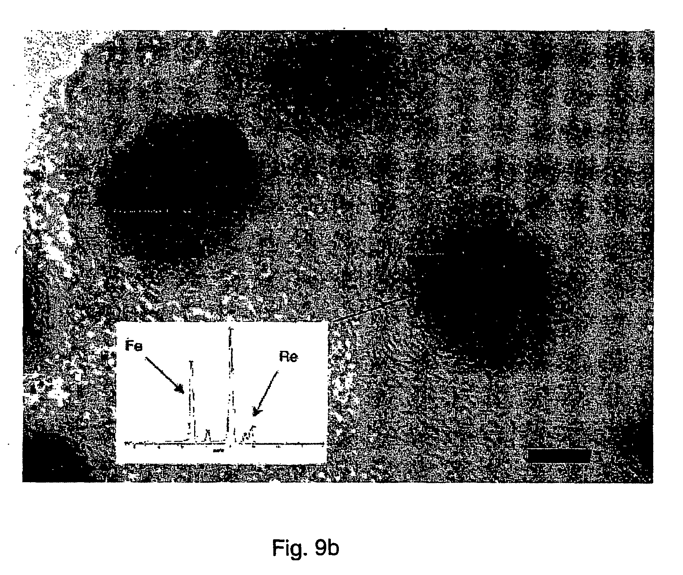

After heat treatment (which may be carried out in various stages or steps) the closed graphitic structures are observed embracing both the iron and rhenium elements within the carbon enclosed shells (Figure 9).

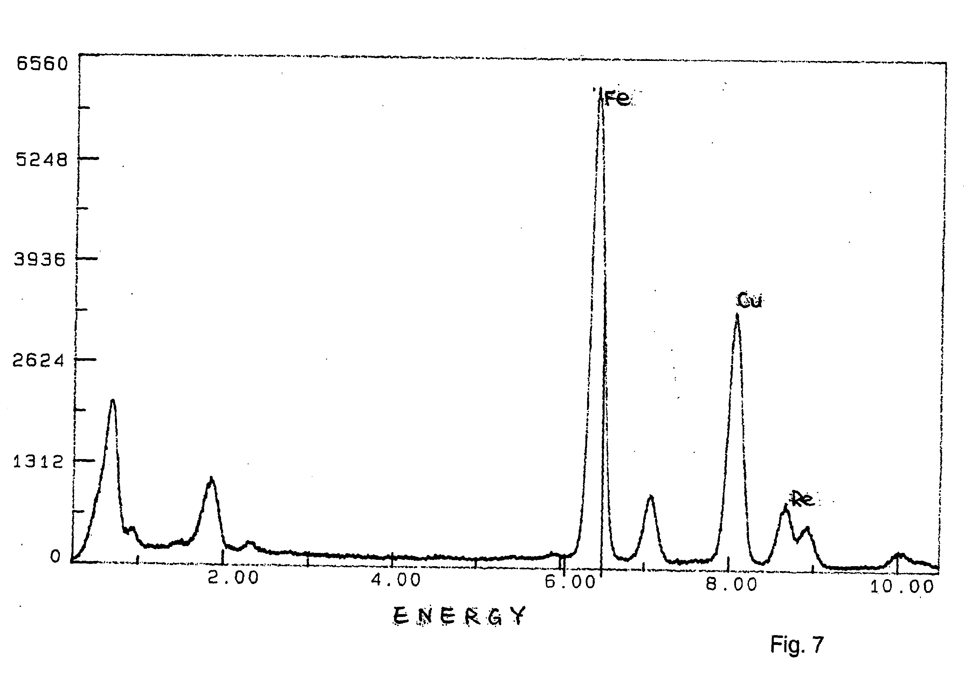

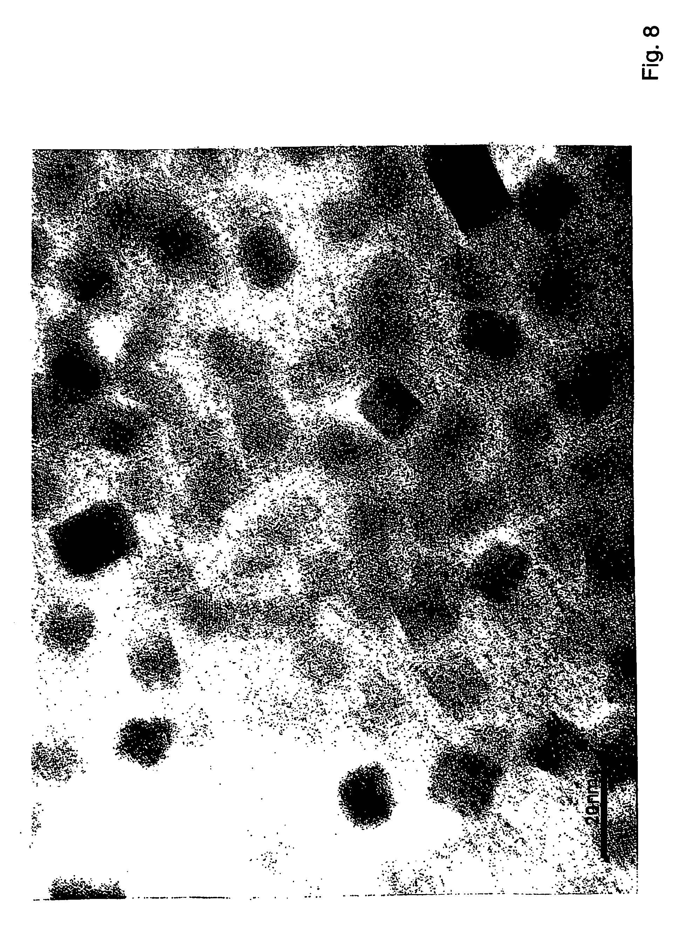

Hence, during the decomposition of iron cyanide at elevated temperature, in the absence of air, the oxygen species will be transferred from the ReO₄⁻ species to the Fe^{II}(CN)₆⁴⁻ (Re(+7) will be reduced while simultaneously oxidising the Fe²⁺) leading to incorporation of the Re species into the stabilised very well-defined, nano-sized iron (oxo) cyanide particles (see EDX analysis in Figure 7 and the TEM micrograph in Figure 8 prior heat treatments).

Because of the d- electron configuration (lanthanide contraction) rhenium (Re) shows almost identical chemical properties as technetium (Tc). Typically, the reduction potentials of the ReO₄⁻ and ^{99m}TcO₄⁻ species are about 0.7 V (V versus NHE). It is reported that a fast rate of reductive deposition of TcO₄⁻ is obtained over small solid magnetite (iron oxide). ^{99m}TcO₄⁻, through its reductive deposition onto the organic stabilised nano-iron containing particles as described in this process, followed by heat treatment, will make a carbon encapsulated particle containing radioactive ^{99m}Tc element. As a result, the small but defined dimensions of the iron core (as a catalyst) can incorporate a significant amount of radioactive ^{99m}Tc into the final carbon encapsulated particles for imaging, storage and radio-therapeutic applications by this technique.

### Example 4 : Synthesis of Carbon Encapsulated Nanoparticles containing Iron and Technetium:

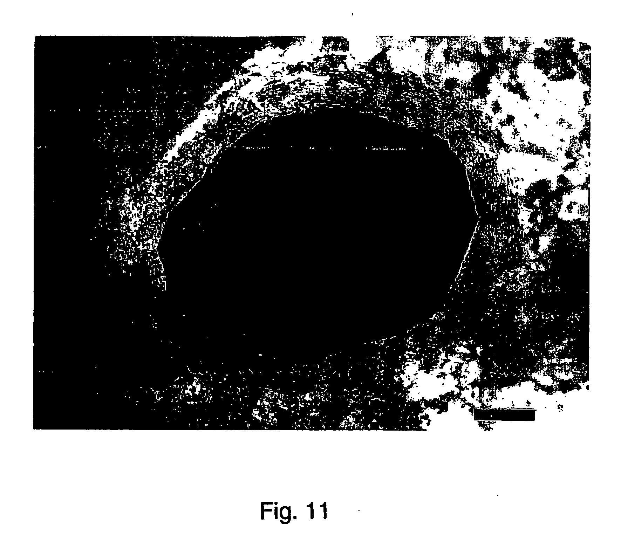

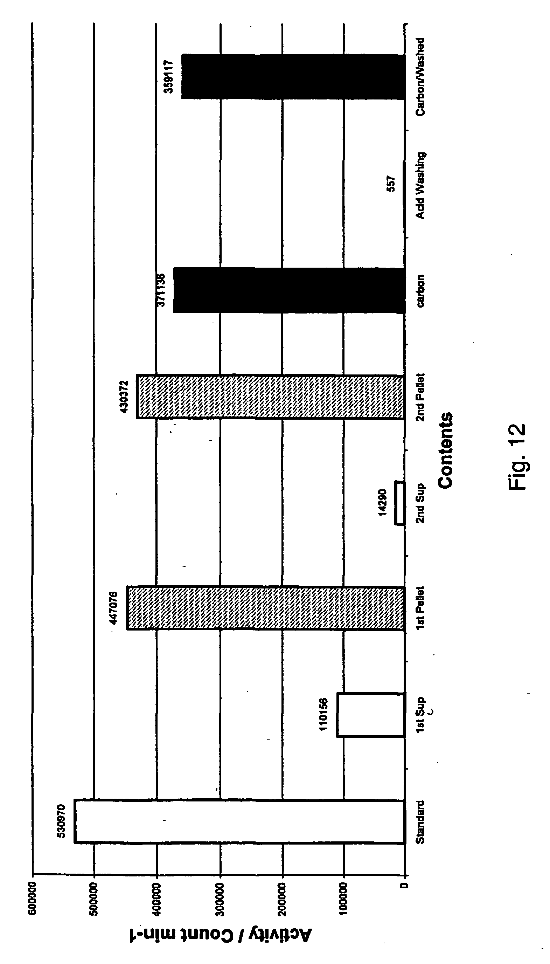

The procedure of Example 3 was repeated, using Na⁹⁹TcO₄ in place of NaReO₄. The technetium was incorporated into the colloidal particles formed and appeared in the pyrolyzed graphitic shell particles. Fig. 11 is a TEM image of the spherical graphitic shell nanoparticles filled with Fe and ^{99m}Tc in the final product. Fig. 12 shows activity counts at different stages of the procedure, as now described. Activity assays were evaluated using gamma counter: 1 mL of ^{99m}TcO₄⁻ of 5.19 x 10⁻¹¹ mole dm⁻³ as the standard. This solution added to the mixture as described and allowed to reflux for 1 h (~1 mL water collected with no radioactivity). Ethanol addition lead to precipitation as 1^{st} pellet and the supernatant as 1^{st} Sup. Repeated treatments produced 2^{nd} Sup and 2^{nd} pellet. After 1000°C for 1 h the 2^{nd} pellet produced solid as carbon. This sample washed with 4M HNO₃, the supernatant as Acid Washing and the solid as Carbon Washed. The radioactivity assays clearly suggested that there had been 13% loss radioactivity due to the physical transfers (powder sticking on reactor tubes), only 1% loss was due to extensive acid treatment (this standard acid digest with sonication would remove externally bound ^{99m}Tc, if any). As a result, the overall radioactivity retention is about 77% in the non-acid leachable solid sample. The surprisingly low activity in the acid wash solution suggests that majority of the particles were chemically protected with acid impenetrable graphitic jackets as indicated from the TEM micrographs.

### Characterisation of the Product:

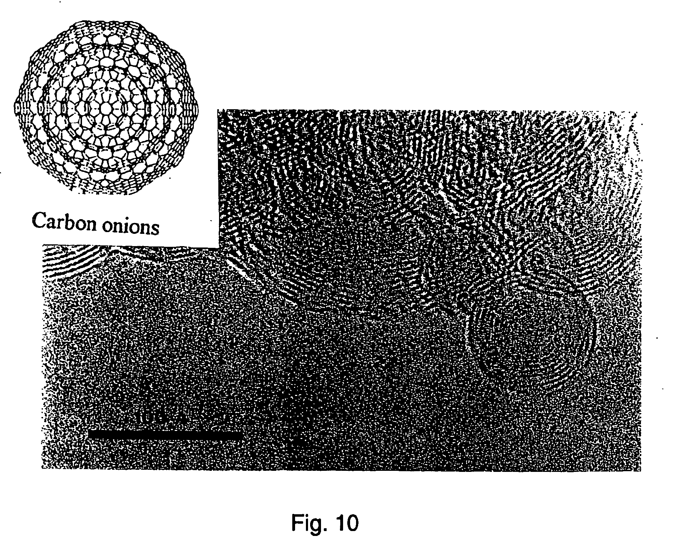

High resolution TEM micrographs indicate that the iron-containing particles were mainly single nano-crystals of γ-Fe metal(confirmed by XRD) although Fe₃C crystals as the inner cores were sometimes observed from the TEM lattice imaging. Detailed examination of the quasi-spherical carbon structures showed that in many cases, carbon lattice fringes (about 0.34 nm) could be traced, continuously yielding, quite surprisingly, hollow concentric carbon shelled structures. No prolonged exposure of the selected area to the electron beam was ensured (<60 seconds); hence the possibility of onions structure formation due to electron beam illumination is rejected. We found some complete filling but more frequently partial filling of the encapsulated iron particles to the carbon cages and there was no obvious preferred lattice fringe orientation with respect to the particle and the carbon layers. It is interesting to note that these highly ordered quasi-spherical concentric graphitic shells structures produced from iron cyanide decomposition are clearly different from tubular or polyhedral carbon structures. Formation of tubular/filamental graphitic carbons is well known to occur when a carbon source (i.e. hydrocarbon gas) is in contact with iron particles at high temperatures [15]. Polyhedral nanoparticles of central cavities of varying sizes were also observed when amorphous carbons were exposed to high temperatures [16]. It is also noted that extremely small quantities of γ-Fe nano-metal crystallites encapsulated in polyhedral nanoparticles [17] were produced in a large amount of carbonanceous debris using the 'stuffed anode' modified Krätschmer-Huffman method where significant quantities of carbon nanotubes or 'sea urchin' structures [18] (carbon nanotubes grow radially from the metal nanocrystal) were also found. None of these structures were however seen in our sample. We note that our quasi-spherical graphitic shell structures rather closely resemble to the 'carbon onions' (see present Figure 10) reported by Ugarte who applied an intense electron beam irradiation on carbon nanotubes for the conversion [19], although structurally less perfect than his. The perfect onion is thought to compose of giant fullerenes giving perfect concentric shells with a quasi-spherical structure. Subsequent work [20] demonstrated 'carbon onions' are highly unstable when not being irradiated in an electron beam and will collapse into a disordered, quite often faceted, configuration, though still with a spheroidal structure somewhat akin to the structures observed.

Here, we have shown that heating the iron(oxo) cyanide nanoparticles (with or without foreign atoms) at 900°C results in graphitisation and the formation of many iron containing nanoparticles encapsulated in quasi-spherical graphitic shell. Presumably, the iron nanoparticles were formed from the reduction of iron oxide with the nearby cyanide source during the carbonisation process.

The phenomenon of 'catalytic' graphitisation is a well-known one [11], but the mechanism in which graphitisation is promoted by the presence of a second phase is not well understood. In some cases, it is believed that the carbon is dissolved in the metal or metal carbide and re-precipitated as graphite. In other cases the metal or metal carbide particles may simply act as templates for the epitaxial growth of graphite. However, in all these cases, only long graphitic tubular or filamental forms of carbons and polyhedral nanoparticles are formed. The exclusive formation of highly ordered quasi-spherical graphitic nanocapsule structures indicative of 'fullerene-like structure' in our case however, has not been reported. It is believed that some nitrogen (residue of cyanide decomposition) resulting from the decomposition of cyanide species may play an important role in self-assembling the carbon atoms into corresponding quasi-spherical structure under our reaction conditions. Nitrogen (non-sp2) adopts different structure from carbon (sp2). Hence its incorporation into carbon graphene layer (all C is in sp2 hybridization) is thought to be responsible for the curvature of the graphitic planes resulting in the particle encapsulation.

The present method for the formation of enclosed graphitic carbon structure embracing iron nanoparticle at relatively very mild temperatures (900°C) is unprecedented. Thus, iron filled spherical carbon nanocapsules of a very narrow size distribution in macroscopic quantities by the controlled decomposition of cyanide-containing species could be prepared. Prior to pyrolysis, use of surfactant/polymer as stabiliser could provide a fine control of particle size. This is based on the observation that the polymer/surfactant adsorbs at the surface of the newly formed nano-particles through the formation of surface active micellar aggregates so preventing aggregation of nanoparticle in the solvent. The internal space is filled with an inorganic iron (oxo) cyanide nanoparticle aggregates with defined dimensions which are in turn controlled by the micellar dimensions. Further immobilisation of other foreign atoms onto the nanoparticle aggregates can be carried out. Here, we show that elemental Re element or Tc can be incorporated into the final product. Thus, the controlled carbonisation of these iron containing nano-assemblies provides the novel carrier which could open up a new avenue for preparing carbon nanocapsules filled with specific radioactive element(s).

There are considerable benefits of forming total carbon encapsulated radionuclides that are well suited for medical diagnosis and therapeutic purposes. The potential beneficiaries will be to patients with lung disease using the carbon encapsulated ^{99m}Tc-Fe nano-particles described herein. For example, pulmonary embolism is a major cause of morbidity and mortality. It is estimated to account for approximately 21,000 deaths annually in England and Wales and over 200,000 in the USA. Early treatment by anticoagulation could save life, but it can sometimes be hazardous; a reliable means of diagnosis is therefore essential. The new technology for producing diagnostic or therapeutic material could provide rapid and efficient diagnosis suitable for patients with this or other pulmonary disease, hence improving life expectancy and quality.

### References:

- 1.: D. Jones, New Scientist, Nov. 3, 1966.
- 2.: J.R. Heath, S.C. O'Brien, Q. Zhang, Y. Liu, R.F. Curl, H.W. Kroto, F.K. Tittel, and R.E. Smalley, JACS 109. 359. 1985.1.
- 3.: S.A. Majetich, J.O. Artman, M.E. McHenry, N.T. Nuhfer and S.W. Staley, Phys Rev. B 48, 16, 845, 1993.
- 4.: S. Seraphin, D. Zhou and J. Jiao, J. Appl. Phys. 80, 1, 1996.
- 5.: Y. Saito in Carbon Nanotubes: Preparation and Properties, ed. T.W. Ebbesen, CRC Press, 1996.
- 6.: Y. Saito, Carbon 33, 979, 1995.
- 7.: V.P. Dravid et al. Nature 374, 602, 1995.
- 8.: J.R.Heath, S.C. O'Brien, Q. Zhang, Y. Liu, R.F. Curl, H.W. Kroto, F.K. Tittel and R.E. Smalley, JACS 109, 359, 1985.
- 9.: D. Ugarte, Chem. Phys. Lett., 209, 99, 1993.
- 10.: S.C. Tsang, P.J.F. Harris, M.L.H. Green, Nature, 362, 520, 1993.
- 11.: S.C. Tsang, Y.K. Chen, P.J.F. Harris and M.L.H. Freen, Nature, 372, 159, 1994.
- 12.: S.C. Tsang, Z. Guo, Y.K. Chen, M.J.H. Green, H.A.O. Hill, T.W. Hambley and P.J. Sadler, Angrew. Chem. Int. Ed. Engl. 36, 2197, 1997.
- 13.: P.J.F. Harris and S.C. Tsang, Chem. Phys. Lett. 293, 53, 1998.
- 14.: P.J.F. Harris and S.C. Tsang, Carbon 36, 1859, 1998.
- 15.: R.T.K. Baker, P.S. Harris, R.B. Thomas, R.J. Waite, J. of Catal., 30, 86, 1973.
- 16.: P.J.F. Harris and S.C. Tsang, Phiil. Mag. A 76, 667, 1997.
- 17.: J.J. Host, M.H. Teng, B.R. Elliott, J.H. Hwang, T.O. Mason, D.L. Johnson, V.P. Dravid, J. Mater. Res., 12, 1268, 1997.
- 18.: S. Subramoney, R. Ruoff, D.C. Lorents, R. Malhotra, Nature, 366, 637, 1993.
- 19.: D. Ugarte, Nature, 359, 707, 1992.
- 20.: G. Lulli, A. Parisani, G. Mattei, Ultramicroscopy, 60, 187, 1995.
- 21.: 'Biomimetic Materials Chemistry' (ed. S. Mann), VCH Press, New York, 1996, chapter 1 and 4 and references therein.
- 22.: W.M. Burch, P.J. Sullivan and C. McLaren. Nucl. Med. Commun., 1986, 7, 865-871; W.M. Burch, P.J. Sullivan, F.E. Lomas, V.A. Evans, C.J. McLaren, R.N. Arnot. J. Nucl. Med., 1986, 27, 842-846; T.J. Senden, K.H. Moock, J.F. Gerald, W.M. Burch, R.J. Browitt, C.D. Ling and G.A. Heath, J. Nucl. Med., 1997, 38, 1327-133.
- 23.: C.W. Ng, J. Ding, L. Wang, L.M. Gan, C.H. Quek, J. Phys. Chem. A 2000, 104, 8814-8822.
- 24.: E. Boellaard, A.M. van der Kraan, J.W. Geus, Preparation of Catalysis VI - Scientific Bases for the Preparation of Heterogeneous Catalysis, G. Poncelet et al. (Editors), Elsevier, Amsterdam, 1995, p. 931.
- 25.: Chemistry and Physics of Carbon, edited by P.A. Thrower, v1-25, Marcel Dekker, New York, Inc. 1977.

## Claims

1. Method of making microparticles having a metal-containing core encapsulated in a graphitic shell containing hetero atoms, including the steps of
(i) forming, in a liquid medium, colloidal particles containing a first metal in the form of a metal-oxo species, the first metal being selected from Fe, Co, Ni and Pd, the particles being colloidally stabilized by a surfactant and containing in addition to the first metal source material of carbon and the hetero atoms, and
(ii) separating said particles from the liquid medium and pyrolyzing them in inert gas to yield the microparticles having said core and said graphitic shell containing said hetero atoms encapsulating the core.

2. Method according to claim 1 wherein said metal containing core contains at least one phase selected from metal, alloy, metal carbide and metal oxide and, optionally, is ferromagnetic.

3. Method according to claim 1 or 2 wherein in step (i) at least one second metal is incorporated in said colloidal particles and is present in the core of the microparticles obtained.

4. Method according to claim 3 wherein said second metal is a radionuclide.

5. Method according to any one of the preceding claims wherein step (i) is performed by forming a solution of a compound of said first metal in said liquid medium which is a polar solvent and heating said solution in the presence of said surfactant and an oxidizing agent to convert said compound to said metal-oxo species and form said colloidal particles.

6. Method according to claim 5 wherein said oxidizing agent is selected from oxygen and a compound of a second metal which becomes incorporated in the colloidal particles.

7. Method according to any one of claims 1 to 4 wherein step (i) is performed by forming an emulsion having dispersed phase droplets stabilized by said surfactant and containing a dissolved compound of said first metal and oxidizing said compound of said first metal to produce said metal-oxo species and form said colloidal particles.

8. Method according to claim 7 wherein said dispersed phase droplets include a dissolved oxidizing agent which is a compound of a second metal which becomes incorporated in said colloidal particles.

9. Method according to any one of the preceding claims wherein said hetero atoms are selected from N, B, P, S and O.

10. Method according to any one of the preceding claims wherein said metal-oxo species is a metal complex including ligands selected from cyanide, isocyanide, cyanate and isocyanate, thereby acting as a source of carbon and nitrogen as said hetero atom.

11. Microparticles having a metal-containing core encapsulated in a graphitic shell, **characterised in that** said metal-containing core comprises at least one of Fe, Co, Ni and Pd and **in that** said graphitic shell contains chemically bound hetero atoms.

12. Microparticles according to claim 11, wherein said metal-containing core contains, in addition to at least one of Fe, Co, Ni and Pd, a radionuclide.

13. Microparticles according to claim 11 or 12, wherein said metal containing core contains at least one phase selected from metal, alloy, metal carbide and metal oxide and, optionally, is ferromagnetic.

14. Microparticles according to claim 11, 12 or 13, wherein said hetero atoms are selected from N, B, P, S and O.

15. Microparticles according to any one of claims 11 to 13, wherein said hetero atoms are N.

## Patentansprüche

1. Verfahren zur Herstellung von Mikroteilchen mit einem metallhältigen Kern, der in eine Graphithülle, die Heteroatome enthält, eingebettet ist, folgende Schritte umfassend:
(i) das Ausbilden, in einem flüssigen Medium, von Kolloidteilchen, die ein erstes Metall in Form einer Metall-Oxo-Spezies enthalten, wobei das erste Metall aus Fe, Co, Ni und Pd ausgewählt ist und die Teilchen mithilfe eines Tensids kolloidal stabilisiert sind und neben der ersten Metallquelle ein Material aus Kohlenstoff und Heteroatomen enthalten, und
(ii) das Trennen der Teilchen vom flüssigen Medium und ihre Pyrolyse in einem Inertgas, um die Mikroteilchen mit dem Kern und der die Heteroatome enthaltenden Graphithülle, in die der Kern eingebettet ist, zu erhalten.

2. Verfahren nach Anspruch 1, worin der metallhältige Kern zumindest eine aus einem Metall, einer Legierung, einem Metallcarbid und einem Metalloxid ausgewählte Phase enthält und gegebenenfalls ferromagnetisch ist.

3. Verfahren nach Anspruch 1 oder 2, worin in Schritt (i) zumindest ein zweites Metall in die Kolloidalteilchen inkorporiert wird und im Kern der erhaltenen Mikroteilchen enthalten ist.

4. Verfahren nach Anspruch 3, worin das zweite Metall ein Radionuklid ist.

5. Verfahren nach einem der vorangegangenen Ansprüche, worin Schritt (i) durchgeführt wird, indem eine Lösung einer Verbindung des ersten Metalls im flüssigen Medium, das ein polares Lösungsmittel ist, gebildet wird und die Lösung in Gegenwart des Tensids und eines Oxidationsmittels erhitzt wird, um die Verbindung in die Metall-Oxo-Spezies zu überführen und die Kolloidteilchen zu bilden.

6. Verfahren nach Anspruch 5, worin das Oxidationsmittel aus Sauerstoff und einer Verbindung eines zweiten Metalls, das in die Kolloidteilchen inkorporiert wird, ausgewählt ist.

7. Verfahren nach einem der Ansprüche 1 bis 4, worin Schritt (i) durchgeführt wird, indem eine Emulsion mit einer dispersen Phase in Form von Tröpfchen gebildet wird, die mithilfe des Tensids stabilisiert ist und eine gelöste Verbindung des ersten Metalls enthält, und die Verbindung des ersten Metalls oxidiert wird, um die Metall-Oxo-Spezies herzustellen und die Kolloidteilchen zu bilden.

8. Verfahren nach Anspruch 7, worin die disperse Phase in Form von Tröpfchen ein gelöstes Oxidationsmittel umfasst, das eine Verbindung eines zweiten Metalls ist, das in die Kolloidteilchen inkorporiert wird.

9. Verfahren nach einem der vorangegangenen Ansprüche, worin die Heteroatome aus N, B, P, S und O ausgewählt sind.

10. Verfahren nach einem der vorangegangenen Ansprüche, worin die Metall-Oxo-Spezies ein Metallkomplex ist, der aus Cyanid, Isocyanid, Cyanat und Isocyanat ausgewählte Liganden umfasst und so als Quelle für Kohlenstoff und Stickstoff als Heteroatom agiert.

11. Mikroteilchen mit einem metallhältigen Kern, der in eine Graphithülle eingebettet ist, **dadurch gekennzeichnet, dass** der metallhältige Kern zumindest eines aus Fe, Co, Ni und Pd umfasst und dass die Graphithülle chemisch gebundene Heteroatome enthält.

12. Mikroteilchen nach Anspruch 11, worin der metallhältige Kern neben zumindest einem aus Fe, Co, Ni und Pd noch ein Radionuklid enthält.

13. Mikroteilchen nach Anspruch 11 oder 12, worin der metallhältige Kern zumindest eine aus einem Metall, einer Legierung, einem Metallcarbid und einem Metalloxid ausgewählte Phase enthält und gegebenenfalls ferromagnetisch ist.

14. Mikroteilchen nach Anspruch 11, 12 oder 13, worin die Heteroatome aus N, B, P, S und O ausgewählt sind.

15. Mikroteilchen nach einem der Ansprüche 11 bis 13, worin die Heteroatome N sind.

## Revendications

1. Méthode de production de microparticules ayant un coeur contenant du métal encapsulé dans une enveloppe en graphite contenant des hétéroatomes, comprenant les étapes de
(i) former, dans un milieu liquide, des particules colloïdales contenant un premier métal sous la forme d'une espèce de métal oxo, le premier métal étant choisi parmi Fe, Co, Ni et Pd, les particules étant colloidalement stabilisées par un agent tensio-actif et contenant en addition au premier métal, une source de carbone et les hétéroatomes, et
(ii)séparer lesdites particules du milieu liquide et les pyrolyser dans un gaz inerte pour donner les microparticules ayant ledit coeur et ladite enveloppe en graphite contenant lesdits hétéroatomes encapsulant le coeur.

2. Méthode selon la revendication 1 où ledit coeur contenant du métal contient au moins une phase sélectionnée parmi un métal, un alliage, un carbure de métal et un oxyde de métal, et, facultativement, est ferromagnétique.

3. Méthode selon la revendication 1 ou 2 où, à l'étape (i), au moins un second métal est incorporé dans lesdites particules colloïdales et est présent dans le coeur des microparticules obtenues.

4. Méthode selon la revendication 3 où ledit second métal est un radionuclide.

5. Méthode selon l'une quelconque des revendications précédentes où l'étape (i) est accomplie en formant une solution d'un composé dudit premier métal dans ledit milieu liquide qui est un solvant polaire et en chauffant ladite solution en présence dudit agent tensio-actif et d'un agent oxydant pour convertir ledit composé en ladite espèce de métal oxo et former lesdites particules colloïdales.

6. Méthode selon la revendication 5 où ledit agent oxydant est sélectionné parmi l'oxygène et un composé d'un second métal qui se trouve incorporé dans les particules colloïdales.

7. Méthode selon l'une quelconque des revendications 1 à 4 où l'étape (i) est accomplie en formant une émulsion ayant des gouttelettes en phase dispersée stabilisées par ledit agent tensio-actif et contenant un composé dissous dudit premier métal et en oxydant ledit composé dudit premier métal pour produire ladite espèce de métal oxo et former lesdites particules colloïdales.

8. Méthode selon la revendication 7 où lesdites gouttelettes en phase dispersée contiennent un agent oxydant dissous qui est un composé d'un second métal qui se trouve incorporé dans lesdites particules colloïdales.

9. Méthode selon l'une quelconque des revendications précédentes où lesdits hétéroatomes sont sélectionnés parmi N, B, P, S et O.

10. Méthode selon l'une quelconque des revendications précédentes où ladite espèce de métal oxo est un complexe de métal comprenant des ligands sélectionnés parmi cyanure, isocyanure, cyanate et isocyanate, pour ainsi servir de source de carbone et d'azote en tant que ladite source d'hétéroatome.

11. Microparticules ayant un coeur contenant du métal encapsulé dans une enveloppe en graphite, **caractérisées en ce que** ledit coeur contenant du métal comprend au moins l'un de Fe, Co, Ni et Pd et **en ce que** ladite enveloppe en graphite contient des hétéroatomes chimiquement liés.

12. Microparticules selon la revendication 11, où ledit coeur contenant du métal contient , en plus d'au moins l'un de Fe, Co, Ni et Pd, un radiionuclide.

13. Microparticules selon la revendication 11 ou 12, où ledit coeur contenant du métal contient au moins une phase sélectionnée parmi un métal, un alliage, un carbure de métal et un oxyde de métal et, facultativement, est ferromagnétique.

14. Microparticules selon la revendication 11, 12 ou 13, où lesdits hétéroatomes sont sélectionnés parmi N, B, P, S et O.

15. Microparticules selon l'une quelconque des revendications 11 à 13, où lesdits hétéroatomes sont N.
